(19) Europäisches Patentamt
European Patent Office
Office européen des brevets

(11) Veröffentlichungsnummer: **0 073 394**
**A1**

(12) # EUROPÄISCHE PATENTANMELDUNG

(21) Anmeldenummer: **82107421.8**

(22) Anmeldetag: **16.08.82**

(51) Int. Cl.³: **C 07 D 233/52**, A 61 K 31/415

(30) Priorität: **27.08.81 DE 3133886**

(43) Veröffentlichungstag der Anmeldung: **09.03.83**
**Patentblatt 83/10**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Behner, Otto, Dr., In den Birken 83, D-5600 Wuppertal 1 (DE)**
Erfinder: **Schütz, Siegismund, Dr., Stintenbergerstrasse 54, D-4020 Mettmann 1 (DE)**
Erfinder: **Stendel, Wilhelm, Dr., In den Birken 55, D-5600 Wuppertal 1 (DE)**
Erfinder: **Andrews, Peter, Dr., Gellertweg 2, D-5600 Wuppertal 1 (DE)**

(54) **2-Arylazo-2-imidazoline, Acylderivate derselben, Verfahren zu ihrer Herstellung und die Verwendung zur Bekämpfung von Ekto- und/oder Endoparasiten.**

(57) Imidazolinverbindungen der Formel

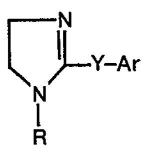

(I)

in der Y für –N=N– steht, Ar einen disubstituierten oder mit einem weiteren Ring anellierten Phenylrest bedeutet und R für H, $C_1$–$C_4$-Alkyl oder Acyl steht, Verfahren zu ihrer Herstellung und ihre Verwendung zur Bekämpfung von Ektoparasiten und Endoparasiten.

EP 0 073 394 A1

0073394

BAYER AKTIENGESELLSCHAFT 5090 Leverkusen, Bayerwerk

Zentralbereich
Patente, Marken und Lizenzen Ad/Kü-c

# 2-Arylazo-2-imidazoline, Acylderivate derselben, Verfahren zu ihrer Herstellung und die Verwendung zur Bekämpfung von Ekto- und/oder Endoparasiten

Die vorliegende Erfindung betrifft neue 2-Arylazo-2-imidazoline, Acylderivate derselben, Verfahren zu ihrer Herstellung und ihre Verwendung zur Bekämpfung von Ektoparasiten und Endoparasiten.

Im Benzolring substituierte 2-Phenylazo-imidazole mit antidepressiver und anxiolytischer Wirksamkeit sind bekannt (US-PS 4 079 130). Entsprechende 2-Phenylazoimidazoline sind unbekannt.

Es wurde nun gefunden, daß die neuen Imidazoline der Formel I

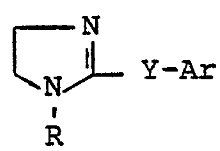

(I)

in der Y für -N=N- steht, Ar einen disubstituierten oder mit einem weiteren Ring anellierten Phenylrest bedeutet und R für Wasserstoff, einen $C_1$-$C_4$-Alkylrest oder Acylrest steht, bzw. deren Salze hervorragend geeignet sind zur Bekämpfung von tierischen Ektoparasiten, insbesondere aus der Klasse der Akariden, sowie von Endoparasiten.

Bei den Acylresten handelt es sich um gegebenenfalls substituierte $C_1$-$C_6$-Alkanoylreste, gegebenenfalls substituierte $C_2$-$C_6$-Alkenoylreste, gegebenenfalls substituierte $C_7$-$C_{10}$-Aroylreste, $C_2$-$C_6$-Alkoxycarbonyl- oder Carbamoylreste, $C_1$-$C_6$-Alkylsulfonylreste oder gegebenenfalls durch $C_1$-$C_2$-Alkylreste, Nitrogruppen der Halogenatome ein- oder mehrfach substituierte $C_6$-$C_{10}$-Arylsulfonylreste.

Bei den Acylresten handelt es sich vorzugsweise um Formyl-, Acetyl-, Trichloracetyl-, Trifluoracetyl-, Propionyl-, Crotonyl-, Benzoyl-, Chlorbenzoyl-, Ethoxycarbonyl-, Methoxycarbonyl-, N-Methylcarbamoxyl-, N-Phenylcarbamoyl-, Methansulfonyl-, Ethansulfonyl-, Benzolsulfonyl-, Chlorbenzolsulfonyl-, Toluolsulfonyl-, Nitrobenzolsulfonyl- oder Naphthalinsulfonylreste.

Bei dem Rest Ar handelt es sich um einen durch $C_1$-$C_3$-Alkyl-, $C_1$-$C_3$-Alkoxy- oder Trifluormethylgruppen oder Halogenatome vorzugsweise in 2,3-Stellung substituiertes Phenyl, um Naphthyl, Tetrahydronaphthyl oder Indanyl.

Weiterhin wurde gefunden, daß man die erfindungsgemäßen Verbindungen der Formel I, in der Y für eine Azogruppe steht, Ar die oben angegebene Bedeutung hat und R für Wasserstoff oder niederes Alkyl steht, erhält, wenn man

a) 2-Arylhydrazino-2-imidazoline der Formel II,

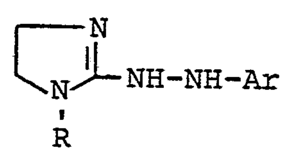

(II)

in der Ar und R die oben angegebene Bedeutungen haben,

dehydriert mit Hilfe von Oxidationsmitteln wie Mennige, Silberoxid, Chromsäure, Wasserstoffperoxid oder auch Luftsauerstoff, oder

b) acyclierte 2-Arylamino-2-imidazoline der Formel III, in der Ar und R die oben angegebenen Bedeutungen haben und R' für einen Alkyl oder (gegebenenfalls substituierten) Phenylrest steht, der Abspaltung von Alkan- oder (substituierter) Benzolsulfinsäure unterwirft.

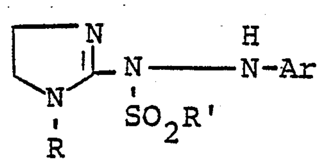

(II)

Die Abspaltung von Sulfonsäure aus den Imidazolinen der Formel III erfolgt zum Teil schon unter den Bedingungen der Synthese dieser Verbindungen; es kann jedoch vorteilhaft sein, die Reaktion bei erhöhter Temperatur durchzuführen.

Die Acylierung der Verbindungen der Formel I, in denen R Wasserstoff bedeutet, zu Verbindungen der Formel I, in denen R ein Acylrest der oben angegebenen Bedeutung ist, wird auf an sich bekannte Weise durchgeführt, beispielsweise indem man die nicht acylierten Verbindungen in einem geeigneten Lösungsmittel oder Lösungsmittelgemisch in Gegenwart eines Säurefängers mit Sulfonsäurehalogeniden, Carbonsäurehalogeniden oder -anhydriden,

0073394

oder Chlorameisensäurehalogeniden oder -anhydriden zur Reaktion bringt oder mit Isocyanaten in Abwesenheit einer Base reagieren läßt. Als Säurefänger eingesetzt Basen können sein z.B. Alkali- oder Erdalkali-hydroxide, -carbonate oder -hydrogencarbonate oder tertiäre Amine wie Triethylamin oder Pyridin.

Bevorzugt betrifft die Erfindung die nachstehend beispielhaft erwähnten Verbindungen.

2-(2,3-Dimethylphenylazo)-2-imidazolin
2-(3-Chlor-2-methylphenylazo)-2-imidazolin
2-(2,3-Dichlorphenylazo)-2-imidazolin
2-(2-Methoxy-3-methylphenylazo)-2-imidazolin
2-(2-Methyl-3-trifluormethylphenylazo)-2-imidazolin
2-(1-Naphthylazo)-2-imidazolin
2-(5,6,7,8-Tetrahydro-1-naphthylazo)-2-imidazolin
2-(4-Indanylazo)-2-imidazolin
2-(2,3-Dimethylphenylazo)-1-methyl-2-imidazolin
2-(1-Naphthylazo)-1-methyl-2-imidazolin
2-(2,3-Dimethylphenylazo)-1-phenylsulfonyl-2-imidazolin
2-(2,3-Dimethylphenylazo)-1-N-methylcarbamoyl-2-imidazolin
2-(2,3-Dimethylphenylazo)-1-methoxycarbonyl-2-imidazolin.

Die neuen Wirkstoffe der allgemeinen Formel sowie gegebenenfalls ihre Salze weisen starke acarizide Wirkung auf, besonders gegen Acariden, die als tierische Ektoparasiten domestizierte Tiere, wie Rinder, Schafe und Kanninchen befallen. Gleichzeitig haben die erfindungsgemäßen Verbindungen nur eine geringe Warmblütertoxizität.

Sie eignen sich daher gut zur Bekämpfung von tierischen Ektoparasiten aus der Klasse der Acariden, Darüber hinaus besitzen sie jedoch auch Wirkung gegen andere Acariden sowie gegen Insekten.

Als wirtschaftlich wichtige Ektoparasiten, die besonders in tropischen und subtropischen Ländern eine große Rolle spielen, seien genannt: die australische und südamerikanische Rinderzecke Boophilus microplus, die südafrikanische Rinderzecke Boophilus decoloratus, beide aus der Familie der Ixodidae, sowie Rinder- und Schafzecken der Genera Rhipicephalus, Amblyomma, Hyalomma.

Im Laufe der Zeit sind insbesondere Zecken gegen die als Bekämpfungsmittel bisher verwendeten Phosphorsäureester und Carbamate resistent geworden, so daß der Bekämpfungserfolg in vielen Gebieten in wachsendem Maße infrage gestellt wird. Zur Sicherung einer wirtschaftlichen Viehhaltung in den Befallsgebieten besteht ein dringender Bedarf an Mitteln, mit denen alle Entwicklungsstadien, also Larven, Nymphen, Metanymphen und Adulti auch resistenter Stämme, beispielsweise des Genus Boophilus sicher bekämpft werden können. In hohem Maße gegen die bisher verwendeten Phosphorsäureester resistent sind beispielsweise in Australien der Mackay-Stamm, der Biarra-Stamm und der Mt-Alford-Stamm von Boophilus microplus.

Die erfindungsgemäßen Wirkstoffe sind sowohl gegen die normalempfindlichen, als auch gegen die resistenten

Stämme, z.B. von Boophilus, gleich gut wirksam. Sie wirken in üblicher Applikation am Wirtstier sowohl direkt auf alle am Tier parasitierenden Formen als auch stark ovizid auf die adulten Formen, so daß der Vermehrungscyclus der Zecken sowohl in der parasitischen Phase auf dem Tier als auch in der nicht parasitären Phase unterbrochen wird. Die Eiablage wird unterbunden, die Entwicklung und das Schlüpfen inhibiert. Hervorzuheben sind insbesondere der schnell eintretende erregende Effekt auf alle parasitierenden Formen, die ihre Saughaltung aufgeben, in unphysiologischer Weise auf dem Wirtstier umherwandern, abfallen und schließlich sterben (detachingeffect), sowie insbesondere auch die gute Wirkung gegen die erfahrungsgemäß schwer bekämpfbaren Metanymphenstadien.

Ferner wirken sie in der gleichen Art auf alle Entwicklungsstadien mehrwertiger Zecken wie z.B. Amblyomma spp., Hyalomma spp., Rhipicephalus spp., Ixodes spp., Hämaphysalis spp., Dermacentor spp.

Ein detaching effect zeigt sich auch bei Insekten, beispielsweise bei Läusen, wie Hämatopinus spp.

Die neuen Verbindungen der Formel I sind darüber hinaus auch gegen Endoparasiten wirksam. Sie wirken vor allem gegen Magen- und Darmnematoden der Wiederkäuer und Fleischfresser, auch solche, die gegen die gebräuchlichen Benzimidazolanthelmintika resistent und damit nicht mehr ausreichend therapierbar sind.

0073394

Sie wirken vorzugsweise gegen Haemonchus in besonders niedriger Dosierung.

Die Wirkung wurde im Tierversuch nach oraler, subcutaner und dermaler Applikation bei stark mit Parasiten befallenen Versuchstieren geprüft. Die angewendeten Dosierungen wurden sehr gut von den Versuchstieren vertragen.

Die Verbindung wirkt nicht nur nach oraler oder parenteraler Verabreichung, sondern auch gut nach dermaler Applikation (Pour-On, Spot-On). Damit werden Verabreichungsweisen möglich, die bei den im Handel befindlichen nur nach oraler Gabe wirkenden Benzimidazol-Anthelmintika nicht möglich sind, andererseits aber für den Anwender vorteilhaft sind wie z.B. die dermale oder subcutane Behandlung.

Die neuen Verbindungen können als Anthelmintika sowohl in der Human- als auch in der Veterinärmedizin verwendet werden. Sie können in bekannter Weise in die üblichen Formulierungen übergeführt werden.

Je nach der vorgesehenen Applikationsform können die neuen Wirkstoffe in die praxisüblichen Formulierungen übergeführt werden, wie beispielsweise Lösungen, Emulsionen, Suspensionen, Pulver, Pasten und Granulate. Diese werden in bekannter Weise hergestellt, z.B.

durch Vermischen der Wirkstoffe mit Streckmitteln, d.h. flüssigen Lösungsmitteln und/oder Trägerstoffen, gegebenenfalls unter Mitverwendung von oberflächenaktiven Mitteln, also Emulgier- und/oder Dispergiermitteln, wobei z.B. im Falle der Verwendung von Wasser als Streckmittel gegebenenfalls organische Lösungsmittel als Hilfslösungsmittel verwendet werden können.

Als Lösungsmittel kommen z.B. infrage; Aromaten (z.B. Xylol, Benzol, Orthodichlorbenzol, Trichlorbenzol), Paraffine (z.B. Erdölfraktionen), Alkohole (z.B. Methanol, Ethanol, Isopropanol, Butanol), stark polare Lösungsmittel wie Dimethylformamid, N-Methyl-pyrrolidon, Dimethylsulfoxid sowie auch Wasser.

Als feste Trägerstoffe seien genannt: natürliche Gesteinsmehle (z.B. Kaoline, Tonerden, Talkum, Kreide) synthetische anorganische Trägerstoffe (z.B. hochdisperse Kieselsäure, Silikate; als Emulgiermittel: sowohl nichtionogene als auch anionische oder kationische Emulgatoren wie z.B. Polyoxyethylen-Fettsäureester, Polyoxyethylen-Fettalkoholether, z.B. Alkylaryl-polyglykolether; Alkylsulfonate und Arylsulfonate, quartäre Ammoniumsalze mit längeren Alkylresten. Als Dispergiermittel seien genannt: Lignin, Sulfitablaugen und Methylcellulose.

Die Formulierungen enthalten im allgemeinen zwischen 0,1 und 95 Gew.-% Wirkstoff, vorzugsweise zwischen 0,5 bis

90 Gew.-%. Die Anwendungskonzentrationen werden aus den Formulierungen (s.o.) durch Verdünnen mit Wasser hergestellt. Sie können, je nach der Anwendungsform, in einem großen Bereich variiert werden und liegen zwischen 1 und 50 000 ppm (g/g), vorzugsweise zwischen 5 und 500 ppm.

Die Applikation erfolgt in üblicher Weise, z.B. durch Besprühen (spray), Gießen (pour on), Vernebeln oder durch Bad (dip).

Den Formulierungen oder den anwendungsfertigen Lösungen können noch sonstige Hilfsmittel oder Wirkstoffe, wie Desinfketionsmittel oder speziell geeignete Insektizide, zugemischt werden.

Die wäßrigen Lösungen bzw. Emulsionen der erfindungsgemäßen Wirkstoffe besitzen unter Praxisbedingungen eine gute Stabilität, so daß die gebrauchsfertigen Anwendungsformen auch bei längerem Stehen und in einem pH-Bereich von 7 - 9 drei Monate und länger wirksam bleiben.

Beispiel 1

2-(2,3-Dimethylphenylazo)-2-imidazolin

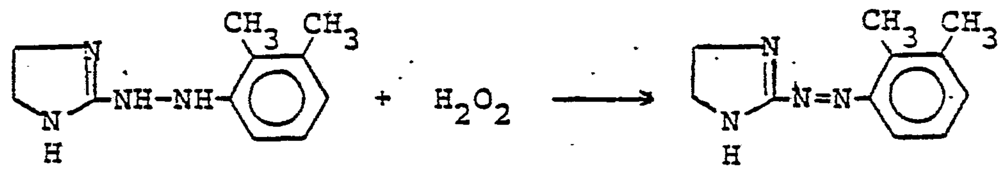

Ein Gemisch aus 3,6 g (0,015 Mol) 2-/2-(2,3-Dimethylphenyl)-hydrazino7-2-imidazolin-hydrochlorid, 50 ml Methylenchlorid und 30 ml 2n Natronlauge wird mit 2 ml 30-proz. Perhydrol versetzt. Man rührt 5 Stunden bei Raumtemperatur, gibt 0,5 ml Perhydrol nach und rührt erneut 1 Stunde, trennt die organische Phase ab, trocknet über Kaliumcarbonat, dampft ein, verreibt den Rückstand mit Hexan und saugt ab. Fp. 115 - 119°, Ausbeute 2,9 g (95,5 % der Theorie).

Die Dehydrierung läßt sich auch mit gleichem Erfolg mit anderen Oxidationsmitteln, z.B. Chromsäure, durchführen.

Beispiel 2

2-(3-Chlor-2-methylphenylazo)-2-imidazolin

Analog Beispiel 1 aus 2-/2-(3-Chlor-2-methylphenyl)hydrazino7-2-imidazolin und Perhydrol. Fp. 125°.

Beispiel 3

2-(2,3-Dichlorphenylazo)-2-imidazolin

Analog Beispiel 1 aus 2-/2-(2,3-Dichlorphenyl)hydrazino/-2-imidazolin und Perhydrol. Fp. 124 - 125°.

Zeckentest

Lösungsmittel: 35 Gewichtsteile Ethylenglykolmonomethylether
35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

In diese Wirkstoffzubereitungen werden adulte, vollgesogene Zeckenweibchen der Art Boophilus microplus (resistent) eine Minute lang getaucht. Nach dem Tauchen von je 10 weiblichen Exemplaren überführt man diese in Petrischalen, deren Boden mit einer entsprechend großen Filterscheibe belegt ist.

Nach 20 Tagen wird die Wirksamkeit der Wirkstoffzubereitung bestimmt durch Ermittlung der Hemmung der Eiablage gegenüber unbehandelten Kontrollzecken. Die Wirkung drückt man in Prozent aus, wobei 100 % bedeutet, daß keine Eier mehr abgelegt wurden und 0 % besagt, daß die Zecken Eier in normaler Menge ablegten.

Untersuchter Wirkstoff, geprüfte Konzentrationen, getestete Parasiten und erhaltene Befunde gehen aus der folgenden Tabelle hervor.

Tabelle 1

In-vitro-Test auf eiablagehemmende Wirkung an Zecken
(Boophilus microplus, Biarra-Stamm)

| Wirkstoff | 100 % | 50 % |
|---|---|---|
| | Hemmung bei einer Wirkstoffkonzentration in ppm a.i. von | |
| 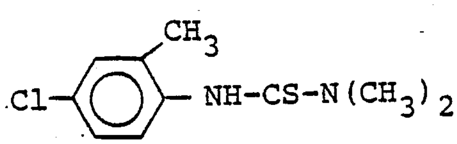 Chloromethiuron (bekanntes Vergleichspräparat) | - | > 5000 |
| 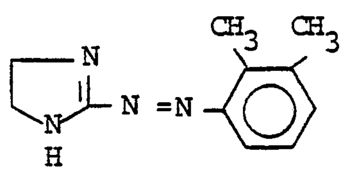 | 100 | 30 |
| 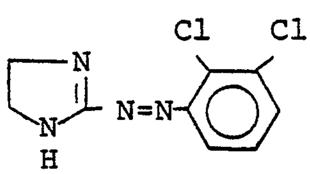 | 3000 | 300 |

Magen- und Darmwurmtest/Schaf

Experimentell mit Haemonchus contortus infizierte Schafe wurden nach Ablauf der Präpatenzzeit der Parasiten behandelt. Die Wirkstoffmenge wurde als reiner Wirkstoff in Gelatinekapseln oral appliziert oder in einem geeigneten Lösungsmittel gelöst und subcutan oder dermal appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren können (Dosis effectiva). Geprüfte Wirkstoffe und wirksame Dosierungen (dosis effectiva minima) sind aus der nachfolgenden Tabelle ersichtlich.

Tabelle 2

| Wirkstoff | Applikationsweise | Dosiseffectiva (Red ≥ 90 % in mg /kg) Haemonchus contortus |
|---|---|---|
| 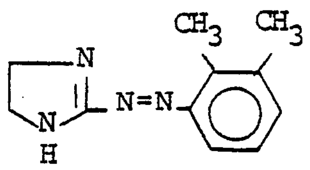 | per os | 0,5 |
| 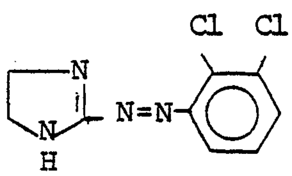 | per os | 1 |
| 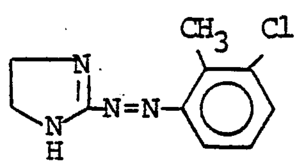 | per os | 0,5 |

## Patentansprüche

1. Imidazolinverbindungen der Formel

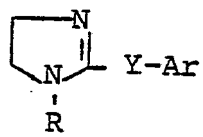

(I)

in der Y für -N=N- steht, Ar einen disubstituierten oder mit einem weiteren Ring anellierten Phenylrest bedeutet und R für H, $C_1$-$C_4$-Alkyl oder Acyl steht.

2. Imidazolinverbindungen nach Anspruch 1, dadurch gekennzeichnet, daß R für einen $C_1$-$C_6$-Alkanoyl-, $C_7$-$C_{10}$-Aroyl-, $C_2$-$C_6$-Alkoxycarbonyl- oder Carbamoylrest, einen $C_1$-$C_6$-Alkylsulfonylrest oder einen gegebenenfalls durch $C_1$-$C_2$-Alkylgruppen, Nitrogruppen oder Halogenatome ein- oder mehrfach substituierten $C_6$-$C_{10}$-Arylsulfonylrest steht und Ar durch $C_1$-$C_3$-Alkyl, Halogen, $C_1$-$C_3$-Alkoxy oder $-CF_3$ disubstituiertes oder mit einem weiteren Ring anelliertes Phenyl bedeutet.

3. Imidazolinverbindungen nach Anspruch 2, dadurch gekennzeichnet, daß der Rest Ar in 2- und 3-Stellung substituiert oder anelliert ist.

4. Verfahren zur Herstellung von Imidazolinverbindungen der Formel

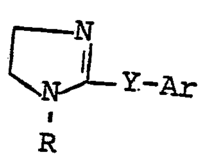

(I)

in der Y für -N=N- steht, Ar einen disubstituierten oder mit einem weiteren Ring anellierten Phenylrest bedeutet und R für H, $C_1$-$C_4$-Alkyl oder Acyl steht, dadurch gekennzeichnet, daß man Verbindungen der Formel

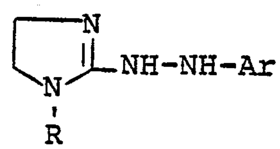

(II)

in der R und Ar die oben angegebene Bedeutung haben, mit Hilfe von Oxidationsmittel dehydriert oder

Verbindungen der Formel

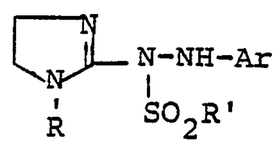

in der R und Ar die oben angegebene Bedeutung haben und R' einen Alkyl- oder Arylrest bedeutet, der Abspaltung des Sulfonylrestes als Sulfinsäure unterwirft.

5. Endo- und/oder ektoparasitizides Mittel, gekennzeichnet durch einen Gehalt an mindestens einer Imidazolinverbindung gemäß Anspruch 1.

6. Anthelmitisches Mittel nach Anspruch 5.

7. Zeckenwirksames Mittel nach Anspruch 5.

8. Verfahren zur Herstellung von endo- und/oder ektoparasitiziden Mitteln, dadurch gekennzeichnet, daß man Imidazolinverbindungen gemäß Anspruch 1 mit inerten, nichttoxischen, pharmazeutisch geeigneten Trägerstoffen vermischt.

9. Verfahren zur Behandlung von Helmithiasen, dadurchgekennzeichnet, daß man Imidazolinverbindungen gemäß Anspruch 1 Menschen oder Tieren appliziert, die an Helmithiasen erkrankt sind.

10. Verwendung von Imidazolinverbindungen gemäß Anspruch 1 in endo- und/oder ektoparasitiziden Mitteln und bei der Bekämpfung von Endo- und/oder Ektoparasiten.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0073394

Nummer der Anmeldung

EP 82 10 7421.8

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | US - A - 4 046 753 (M.H. FISHER et al.)<br>* Zusammenfassung; Spalte 7, Zeilen 14 bis 16; Beispiel 3 * | 1,4,<br>5-7 | C 07 D 233/52<br>A 61 K 31/415 |
| A | GB - A - 2 057 874 (MOCHIDA SEIYAKU K. K.)<br>* Anspruch 1 *<br>& DE- A - 3 028 927<br>-- | 1 | |
| | | | RECHERCHIERTE SACHGEBIETE (Int. Cl.³) |
| D,A | US - A - 4 079 130 (A.H. ABDALLAH et al.)<br>* Beispiele 1, 2 ; Tabelle 1 *<br>-- | 1 | A 61 K 31/415<br>C 07 D 233/00 |
| P,A | EP - A2 - 0 049 797 (BAYER AG)<br>* Ansprüche 1 bis 5 *<br>---- | 3,5,<br>6,10 | |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1 bis 8, 10

Unvollständig recherchierte Patentansprüche:

Nicht recherchierte Patentansprüche: 9

Grund für die Beschränkung der Recherche:

Verfahren zur chirurgischen oder therapeutischen Behandlung des menschlichen oder tierischen Körpers

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| Recherchenort | Abschlußdatum der Recherche | Prüfer |
|---|---|---|
| Berlin | 29-10-1982 | HASS |

EPA Form 1505.1 06.78